# EUROPÄISCHE PATENTSCHRIFT

(11) **EP 0 485 943 B1**
(45) Veröffentlichungstag und Bekanntmachung des Hinweises auf die Patenterteilung: **05.02.1997**
(21) Anmeldenummer: 91119250.8
(22) Anmeldetag: 12.11.1991
(51) Int. Cl.: A61F 5/01

(54) **Knöchelpelotte, Pelotteneinheit und diese enthaltende Knöchelbandage**
Ankle pod, pod unit and ankle bandage containing same
Pelote pour la cheville, chevillère et bandage de la cheville les contenant

(30) Priorität: 13.11.1990 DE 9015508 U
(43) Veröffentlichungstag der Anmeldung: 20.05.1992
(73) Patentinhaber: Kleylein, Horst, D-90429 Nürnberg (DE)
(72) Erfinder: Kleylein, Horst, D-90429 Nürnberg (DE)
(74) Vertreter: Merten, Fritz

(56) Entgegenhaltungen:
- EP-A- 0 350 517
- FR-A- 2 607 383
- GB-A- 740 621
- US-A- 4 705 025
- US-A- 4 825 856

## Beschreibung

Die Erfindung betrifft zunächst eine viskoelastische Knöchelpelotte mit den Merkmalen des Oberbegriffs des Anspruches 1.

Derartige durch eine viskoelastische Profileinlage gebildete Pelotten sind als Bestandteil beispielsweise von Fußgelenkbandagen bekannt. Sie sind dazu bestimmt, einen intermittierenden Kompressionsdruck gezielt auf die Gelenkweichteile auszuüben. Dazu sind sie so ausgestaltet, daß sie nur in den Knöchelfurchen, also nur im peripheren Reliefbereich des Fußknöchels beidseitig den Kompressionsdruck von den Knöchelvorsprüngen ableiten und eine rhythmische Massagewirkung auf Weichteile, Gelenkkapseln und Sehnenlogen ausüben. Die ständig massierende Wirkung der Silikonpuffer soll zur raschen Resorption von Ödemen und Hämatomen die Durchblutung fördern sowie ein rasches Abschwellen, Schmerzlinderung und Funktionsverbesserung bewirken.

Aus der EP-A-0 350 517 ist eine medizinisch orientierte Einlage bekannt, bei der seitlich einer Schuhinnensohle zwei Schäfte mit Pelotten aus einem formelastischen Werkstoff angebracht sind. Die Schäfte sind über je einen Saum mit der Sohle verbunden. Die Pelotten sind scheiben förmig und mit L-förmigen Polstern ausgestaltet. Die Polster umgreifen den Knöchel des Sprunggelenks.

Der Erfindung liegt die Aufgabe zugrunde, eine derartige Pelotte für einen noch breiteren Indikationsbereich weiterzuentwickeln. Diese Aufgabe wird durch die Kennzeichnungsmerkmale des Anspruches 1 gelöst. Diese Pelotte ist bei beidseitiger Anwendung dazu geeignet, das Sprunggelenk zumindest teilweise zu stabilisieren und dabei teilweise sogar eine Gipsruhigstellung überflüssig zu machen. Sie ist in der Lage, entstauend und damit dekomprimierend am Innenknöchelbereich (Tarsaltunnel unter dem Ligamentum laciniatum) zu wirken.

Die Pelotte muß nicht Bestandteil einer Sprunggelenkbandage sein. Sie kann beispielsweise auch bei Bandagierungen angewendet werden. In der Regel wird beidseitig am Fuß je eine Pelotte angelegt, gleichgültig ob dies mit einer Bandagierung oder durch eine vorgefertigte, elastische Bandage erfolgt.

Die Pelotte weist einseitig eine ebene, im Tragezustand fußabseitige Außenseite und eine leicht konvex gewölbte, mit stetig geringer werdender Wandstärke zu den Rändern hin abfallende, im Tragezustand dem Fuß zugewandte Innenseite auf. Dabei ist die jeweilige Wandstärke gerade im Randbereich den Reliefstrukturen, nämlich der Anatomie des Fußes angepaßt. Die Umrißform einer von der linken und einer von der rechten Seite her an den Knöchelbereich eines Fußes anzulegenden Pelotte ist spiegelbildich gleich, nur mit dem Unterschied, daß jeweils die Konvexseite dem Fuß zugewandt ist. Zur wenigstens partiellen Überdeckung auch des Fersenbereiches weist die Pelotte eine zungenartig vorstehende, deutlich erkennbare Ausladung auf.

Durch die erfindungsgemäße Gestaltung der Pelotten fließt deren viskoelastischer Werkstoff je nach Reliefform des Fußes sowie je nach dem beispielsweise von einer Bandagierung oder auch von einer Schuhschnürung ausgeübten Druck mehr oder weniger auch in die den Knöchelbereich und/oder in den Achillessehnenbereich und/oder in den Fersenbereich und/oder in den Sohlenbereich hinein und übt dort Druck auf ein vorhandenes Ödem aus. Dies ist durch die gegenüber bekannten derartigen Pelotten deutlich größere Flächenerstreckung der erfindungsgemäßen Pelotte möglich.

Weil sich die erfindungsgemäße Pelotte beispielsweise auch in den besonders druckempfindlichen Knöchelbereich hinein erstreckt, ist es weiterhin zweckmäßig, sie aus unterschiedlich konsistenten Silikonwerkstoffen derart herzustellen, daß beispielsweise im Knöchelüberdeckungsbereich ein relativ weicher und im Knöchelfurchen- oder Reliefbereich ein vergleichsweise harter Silikonwerkstoff verwendet wird.

Ein weiteres Erfindungsmerkmal sieht in der Pelotte eine Profileinlage aus einem vergleichsweise harten, wenig elastischen Werkstoff zur Stabilisierung im Knochenfurchen- bzw. Reliefbereich vor. Sie ist so gestaltet, daß sie im Anlagezustand der Pelotte im Knochenfurchenbereich des Fußes einliegt, also den Knöchelbereich von hinten und unten gewissermaßen umgibt. Diese Versteifung besteht aus einem L-förmig gestalteten Flächenteil, insbesondere aus einem PVC. Durch diesen Werkstoff ist eine Bindung bzw. Verklebung mit dem Silikonwerkstoff der Pelotte möglich. Die stabilisierende Einlage dient zur Ruhigstellung des Sprunggelenkes und gewährleistet eine Seitenstabilisierung des Knöchelgelenkes. Dadurch, daß sie im Knöchelfurchenbereich einliegt, ist dort von Hause aus ohnehin eine Massierung des fließfähigen Silikonwerkstoffes vorhanden. Durch die Einlage der Versteifung wird die Fließfähigkeit des Silikonwerkstoffes gedämpft. Dadurch wird die Ausübung eines Anpreßdruckes seitens einer Bandage, einer Bandagierung oder eines Schnürschaftes begünstigt. Das wiederum sorgt für einen großflächig gleichbleibenden Anpreßdruck, der - wie gewünscht - auch wirksam ist im Knöchel-, Achillessehnen-, Fersen- und Fußsohlenbereich.

Die Verwendung einer derartigen, L-förmigen, im Tragezustand in der Knöchelfurche einliegenden Versteifungseinlage ist auch bei einer Pelotte herkömmlicher Umrißform vorteilhaft, die mit ihrem viskoelastischen Werkstoff nur innerhalb der Knöchelfurche bzw. des Reliefbereiches des Fußes einliegt.

Als Pelottenwerkstoff ist vorteilhaft ein Additions-Silikon verwendet.

Durch ein weiteres Erfindungsmerkmal sind zwei Sprunggelenkpelotten im Bereich ihrer fußsohlenseitigen Kanten durch einen dünnwandigen Steg miteinander verbunden. Dieser Steg erstreckt sich zweckmäßig über mindestens ein Drittel der Länge der fußsohlenseitigen Pelottenkante. Diese Ausgestaltung hat mehrere Vorteile. Zum einen bindet sie beide üblicherweise gemeinsam zur Anlage gelangenden Pelotten aneinander, so daß eine höhere Verliersicherheit gegeben ist. Darüber hinaus erleichtert sie das Anlegen am Fuß bei der Bandagierung. Bei der Einlage in einer anatomisch formgestreckten Zweizugbandage in Form gewissermaßen eines elastischen Strickschlauches hat der Verbindungssteg den Vorteil, daß dieselbe Bandage mit den Pelotten in gleicher Weise für eine Verwendung am linken wie am rechten Fuß in Betracht kommt, während bei solchen herkömmlichen Knöchelbandagen wegen der unterschiedlichen Höhenlage des Innen- und des Außenknöchels am menschlichen Fuß jeweils gesondert linke und rechte Knöchelbandagen erforderlich waren. Der dünnwandige, elastische Verbindungssteg vermag auch durch die Beaufschlagung der Fußsohle eine Abroll- und Auftrittdämpfung sowie eine Korrektur von Rückfuß- und Mittelfußstellung auszuüben. Damit bedeutet dieser Verbindungssteg eine weitere therapeutische Bereicherung. Auch die Anwendung dieses Verbindungssteges zwischen beiden Pelotten ist nicht zwangsläufig an die Pelottenform gemäß Anspruch 1 gebunden. Vielmehr kann dieser Verbindungssteg auch vorteilhaft bei herkömmlichen, im Anlegezustand nur die Knöchelfurche ausfüllenden Sprunggelenkpelotten verwendet werden.

Die Erfindung wird anhand von in den Figuren dargestellten Ausführungsbeispielen näher erläutert. Es zeigen:
- Fig. 1: eine schematische Fußdarstellung mit angelegter Pelotte nach der Erfindung;
- Fig. 2: eine Ansicht der Fußrückseite mit beidseitig anliegenden Pelotten;

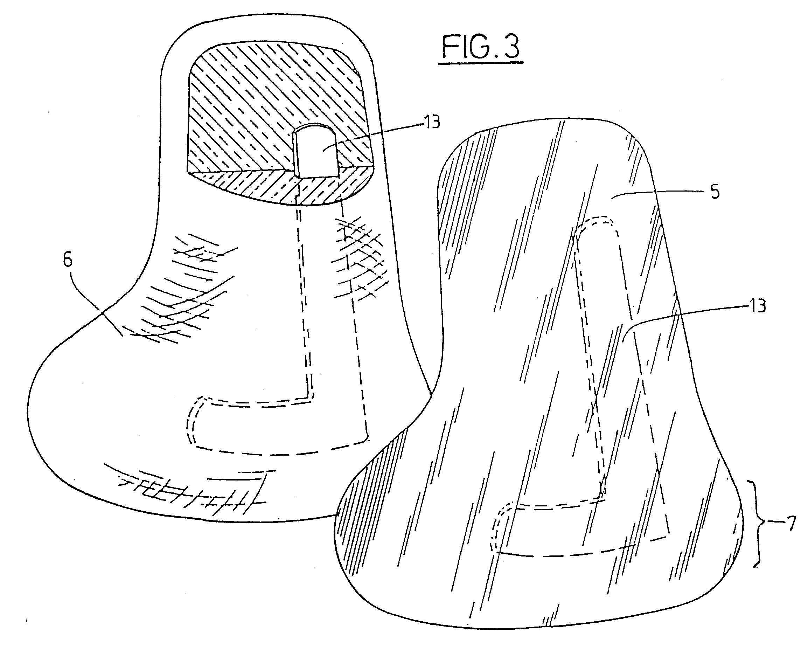

- Fig. 3: in gemeinsamer Perspektivstellung zwei erfindungsgemäß ausgestaltete Pelotten mit entsprechend der beiderseitigen Anlagestellung am Fuß einander zugewandten Konvexseiten;

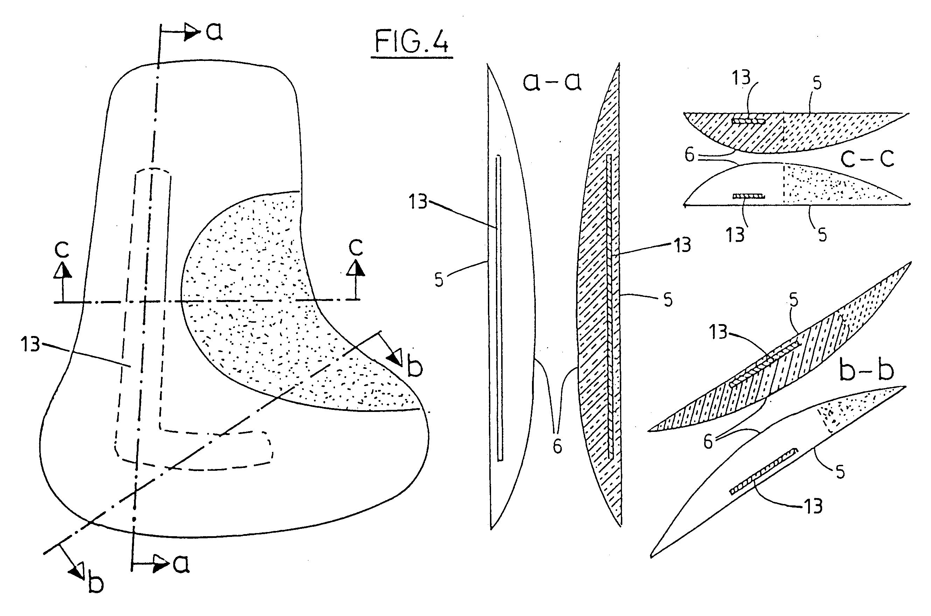

- Fig. 4: die Seitenansicht einer Pelotte mit Schnittdarstellungen aa-aa, bb-bb und cc-cc in unterschiedlichen Schnittrichtungen und mit jeweils einmal linker und einmal rechter Konvexseite;

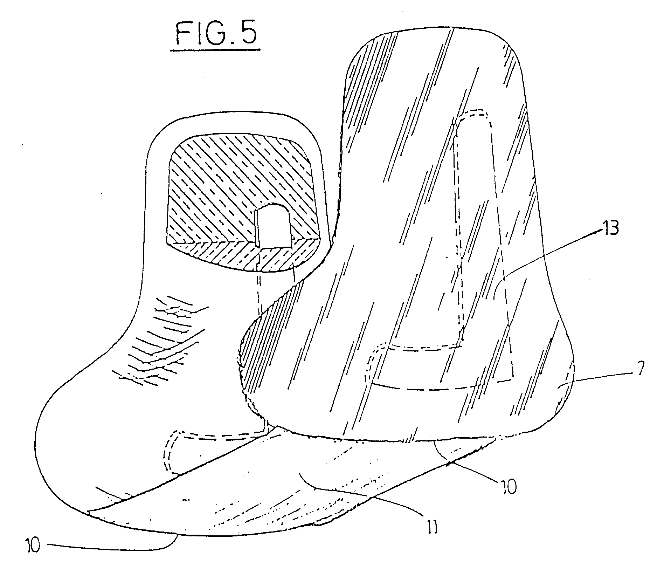

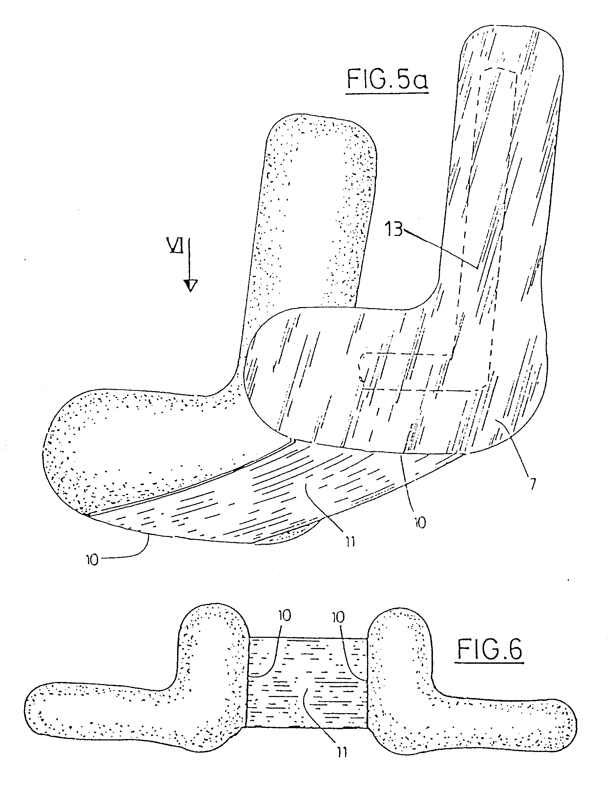

- Fig. 5: zwei Pelotten mit im Bereich ihrer fußsohlenseitigen Randkanten angeordnetem, dünnwandigem Verbindungssteg;
- Fig. 5a: eine Pelotteneinheit analog Fig. 5 jedoch mit einer abgewandelten Umrißform der Pelotten;
- Fig. 6: eine Draufsicht auf die Pelotteneinheit entsprechend Pfeil VI in Fig. 5a mit in die Ebene des Verbindungssteges auseinandergeklappten Pelotten;

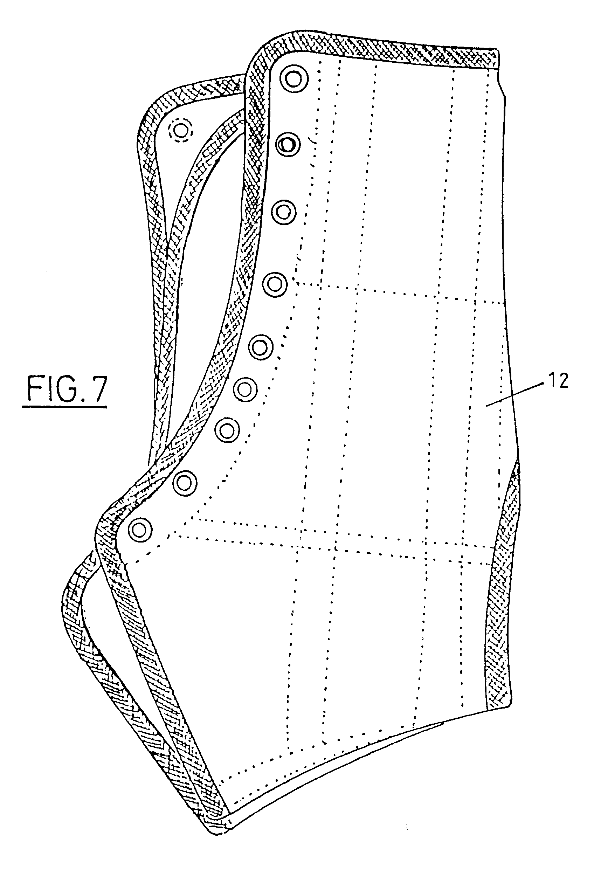

- Fig. 7: die Ansicht einer nach Art eines Schuhschaftes schnürbaren Bandage mit - von außen nicht erkennbar - auf der Innenseite vorliegender Applikation von Pelotten gemäß Fig. 1 - 6.

Die viskoelastische Profileinlage zur Erzeugung eines intermittierenden Kompressionsdruckes im Sprunggelenkbereich, nämlich die Knöchelpelotte, ist gekennzeichnet durch eine großflächige Ausbildung derart, daß sie neben dem Knochenfurchen- bzw. neben dem Reliefbereich zwischen Knöchel einerseits und Achillessehnen-, Fersen-, und Fußsohlenbereich andererseits auch noch den Knöchelbereich 1 des Fußes selbst und/oder dessen Achillessehnenbereich 2, und/oder dessen Fersenbereich 3 und/oder dessen Fußsohlenbereich 4 wenigstens partiell abdeckt. Dazu weist die Pelotte einseitig eine ebene, im Tragezustand fußabseitige Außenseite 5 auf. Anderseitig ist eine leicht konvex gewölbte, mit stetig geringer werdender Wandstärke zu den Rändern hin abfallende und im Tragezustand dem Fuß zugewandte Innenseite 6 vorhanden. In ihrem den Fersenbereich 3 des Fußes partiell abdeckenden Bereich weist die Pelotte eine zungenartig vorstehende Ausladung 7 auf.

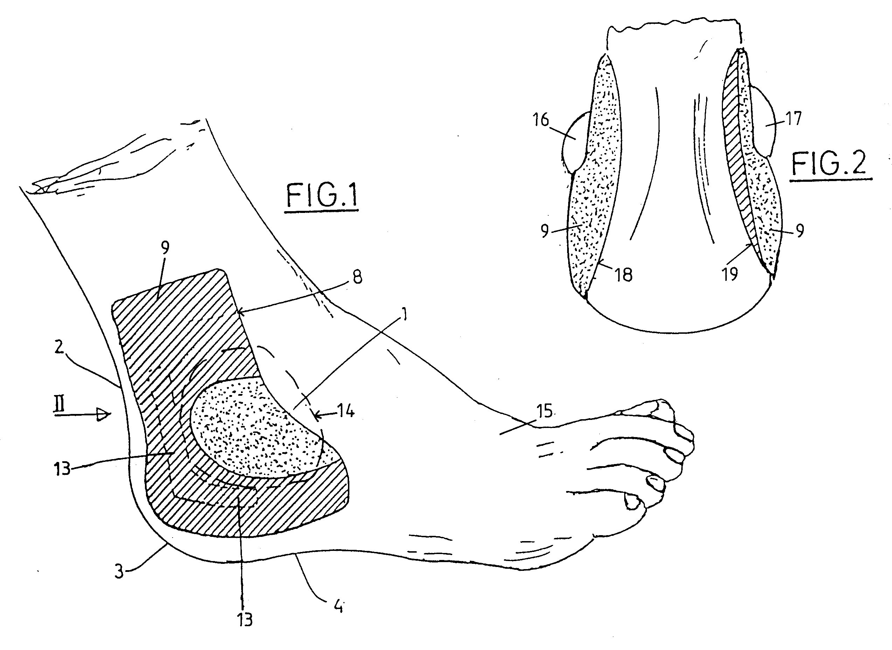

Die Pelotte ist aus unterschiedlich konsistenten Silikonwerkstoffen gebildet. Im Knöchelbereich 1 ist beispielsweise ein in Fig. 1 punktiert dargestellter, vergleichsweise weicher Silkonwerkstoff verwendet, der sich durch eine leichtere Gleit-Fließbarkeit auszeichnet, indessen doch einen hinreichenden Druck auf den Knöchelbereich ausübt, jedoch in diesem besonders druckempfindlichen, weil weit vorstehenden Bereich einen leichteren Ausgleich schafft. Im übrigen, in Fig. 1 schraffiert dargestellten Pelottenbereich 8 kommt ein vergleichsweise härterer Silikonwerkstoff zur Anwendung, wo im Tragezustand hauptsächlich der Knochenfurchenbereich des Fußes vorhanden ist.

Eine Besonderheit ist eine vergleichsweise wenig elastische Profileinlage 9 zur Stabilisierung des Reliefbereiches des Fußes. Die Profileinlage weist im wesentlichen eine L-Form zur Einlage im besagten Knöchelfurchen- oder Reliefbereich auf. Sie ist ein dünnwandiger, ebener Formkörper und besteht aus einem PVC. Der Silikonwerkstoff der Pelotte ist ein Additions-Silion.

Eine weitere Besonderheit besteht in der Zusammenfügung zweier zur Anlage am selben Fuß bestimmter Pelotten zu einer Pelotteneinheit dadurch, daß ihre fußsohlenseitigen Kanten 10 durch einen dünnwandigen Steg 11 aus dem Silikonwerkstoff miteinander verbunden sind. Der Steg 11 erstreckt sich über mindestens ein Drittel der Länge der fußsohlenseitigen Pelottenkanten 10.

Zwei Pelotten oder eine aus zwei Pelotten bestehende Pelotteneinheit der vorstehend beschriebenen Art können Bestandteile einer Knöchelbandage aus einer auch anatomisch formgestrickten Zweizugbandage sein. Es ist indessen in gleicher Weise möglich, sie zum Bestandteil einer Knöchelbandage zu machen, die nach Art eines auch über den Knöchel nach oben hinausgezogenen Schnürschuhschaftes ausgestaltet ist.

### Bezugszeichen

- 1: Knöchelbereich
- 2: Achilessehnenbereich
- 3: Fersenbereich
- 4: Fußsohlenbereich
- 5: Außenseite
- 6: Innenseite
- 7: Ausladung von Pelotte
- 8: Pelotte Knöchelpelotte
- 9: Profileinlage
- 10: Kanten
- 11: Steg
- 12: Schnürschuhschaft
- 13: L-förmiges Verstärkungsteil
- 14: Sprunggelenk
- 15: Fuß
- 16: Knöchel
- 17: Knöchel
- 18: Flächen von Profileinlage
- 19: Flächen von Profileinlage
- punktiert: = weicher Silikonbereich
- schraffiert: = härterer Silikonbereich

## Patentansprüche

1. Knöchelpelotte (8) zur Erzeugung eines intermittierenden Kompressionsdruckes im Bereich eines Sprunggelenkes (14) eines Fußes (15), bestehend aus einer aus einem viskoelastischen Werkstoff gefertigten Profileinlage (9) für jede Seite des Sprunggelenkes (14), wobei jede Profileinlage (9) an einem sie haltenden und sie gegen das Sprunggelenk (14) pressenden Spannkörper angebracht ist und die jeweilige Profileinlage den ihr zugewiesenen Knöchel (16, 17) des Sprunggelenkes (14), mindestens partiell abdeckt,
**dadurch gekennzeichnet**,
daß jede Profileinlage (9) der Knöchelpelotte (8) lappenförmig ausgebildet ist und deren zum Sprunggelenk (14) weisenden Flächen (18, 19) konvex gewölbt und neben dem Reliefbereich zwischen Knöchel (16, 17) einerseits und Achillessehnenbereich (2) andererseits, wie auch Fersenbereich (3) und Fußsohlenbereich (4) großflächig, wie auch den Knöchelbereich (1) mindestens partiell abdeckt,
und daß jede Profileinlage (9) zur Ausfüllung der jeweiligen, anatomischen einfallenden Stellen des um den jeweiligen Knöchel (16, 17) vorgesehenen furchenförmigen Bereichs an ihrer zum Sprunggelenk (14) weisenden Seite in diese einfallenden Stellen dieses Bereichs einbettbare Wandteile aufweist, die im Tragezustand der Knöchelpelotte (8) in diesen Stellen eingebettet sind und gegen das Sprunggelenk (14) zur Anlage kommen und dort neben dem Knöchel selbst auch den Bereich um den Knöchel (16, 17) ausgefüllt und komprimiert halten, wie auch dort dem anatomischen Relief des Sprunggelenkes (14) durch eine mehr oder weniger starke Anhäufung des Materials der Profileinlage (9) folgen, in der Art, daß diese Anhäufungen des Materials vom Fersenbereich (3) zum Vorfußbereich hin fallend verläuft.

2. Knöchelpelotte (8) nach Anspruch 1,
**dadurch gekennzeichnet**,
daß jede ihrer Profileinlagen (9) eine, vom Achillessehnenbereich (2) zur Innenseite (6) des Fußes (15) hin abfallendes Relief aufweist, und daß dieses Relief konvex gewölbt und mit seiner konvexen Innenseite (6) zum Sprunggelenk (14) hin gerichtet ist, wie auch dessen nach außen gerichteten Ränder an deren Außenseiten (5) flach werdend, auslaufen.

3. Knöchelpelotte (8) nach den Ansprüchen 1 und 2,
**dadurch gekennzeichnet**,
daß jede Profileinlage (9) an ihren, zum Fersenbereich (3) des Fußes (15) hinreichenden Bereich eine vorstehende Ausladung (7) aufweist.

4. Knöchelpelotte (8) nach Anspruch 1,
**dadurch gekennzeichnet**,
daß der viskoelastische Werkstoff jeder Profileinlage (9) ein Silikonwerkstoff ist und dieser Werkstoff entlang dem Profil der Profileinlage (9), beginnend am Achillessehnenbereich (2) und auslaufend zur Innenseite des Fußes (15) eine unterschiedliche Härte ihres Materials aufweist, in der Art, daß um den Bereich des Knöchels (16, 17) eine geringere Härte, hingegen im restlichen Bereich der Profileinlage (9) eine größere Härte vorgesehen ist.

5. Knöchelpelotte (8) nach Anspruch 4,
**dadurch gekennzeichnet**,
daß eine der beiden Profileinlagen (9) in ihrer Gesamtheit aus einem, eine geringere Härte aufweisenden viskoelastischen Werkstoff hergestellt ist als die andere Profileinlage (9).

6. Knöchelpelotte (8) nach Anspruch 1,
**dadurch gekennzeichnet**,
daß mindestens eine der Profileinlagen (9) zur weiteren Stabilisierung des Sprunggelenkes (14) ein L-förmiges Verstärkungsteil (13) aufweist, welches entlang einer anatomischen Einfallstelle (Furche) zwischen dem Knöchel (16) und dem Fersenbereich (3) vorgesehen ist.

7. Knöchelpelotte (8) nach Anspruch 4,
**dadurch gekennzeichnet**,
daß der viskoelastische Werkstoff jeder Profileinlage (9) ein PVC ist.

8. Knöchelpelotte (8) nach Anspruch 4,
**dadurch gekennzeichnet**,
daß der viskoelastische Werkstoff jeder Profileinlage (9) ein Additions-Silikon ist.

9. Knöchelpelotte (8) nach einem oder mehreren der Ansprüche 1-8,
**dadurch gekennzeichnet**,
daß beide Profileinlagen (9) an ihren jeweiligen, im Fußsohlenbereich (4) liegenden Kanten (10) über einen Steg (11) miteinander verbunden sind, und daß dieser Steg, im Tragezustand der Knöchelpelotte (8) unterhalb der Sohle zwischen Fersenbereich (3) und Fußballen liegt.

10. Knöchelpelotte (8) nach Anspruch 9,
**dadurch gekennzeichnet**,
daß der Steg (11) sich über mindestens ein Drittel der Länge der fußsohlenseitigen Kante (10) der Profileinlage (9) erstreckt.

11. Knöchelpelotte (8) nach mindestens einem der Ansprüche 1-10, wobei die Profileinlagen (9) von einer elastischen, formgestrickten Bandage um den jeweiligen Knöchel (16, 17) des Sprunggelenkes (14) gehalten wird,
**dadurch gekennzeichnet**,
daß die Kompression deren Wandteile (Flächen 18, 19) auf das Sprunggelenk (14) von dem Grad der Elastizität der Bandage abhängig ist.

12. Knöchelpelotte (8) nach mindestens einem der Ansprüche 1-10,
**dadurch gekennzeichnet**,
daß die Profileinlagen (9) an den inneren Seiten eines halbstiefelförmigen Schaftes (12) angebracht sind und daß die Kompression deren Wandteile (Flächen 18, 19) auf das Sprunggelenk (14) vom Grad der Schnürung dieses Schaftes abhängig ist.

## Claims

1. Ankle pad (8) for producing an intermittent compression in the area of an ankle joint (14) of a foot (15), consisting of a profiled inlay (9) made of a viscoelastic material for either side of the ankle joint (14), wherein each profiled inlay (9) is fastened on a tensioning element holding it and pressing it against the ankle joint (14) and the respective profiled inlay covers, at least partially, the ankle (16, 17) associated to it of the ankle joint (14),
**characterized in that**
each profiled inlay (9) of the ankle pad (8) is shaped like a tab and its surfaces (18, 19) facing the ankle joint (14) have a convex curvature and cover, in addition to the relief area between the ankle (16, 17) on the one hand and the Achilles tendon area (2) on the other hand, the heel area (3) and the sole area (4) over a large surface, as well as, at least partially, the ankle area (1),
and in that each profiled inlay (9) possesses wall parts imbeddable in the inlay on its side facing the ankle joint (14) in the respective anatomically recessed portions of the furrow-shaped area around the respective ankle (16, 17) in order to fill these recessed portions of this area, said wall parts being embedded in these portions when the ankle pad (8) is being worn and contacting the ankle joint (14) and keeping there the ankle itself as well as the area around the ankle (16, 17) filled and compressed, and in addition following there the anatomic relief of the ankle joint (14) through a more or less substantial accumulation of the material of the profiled inlay (9), in such a way that these accumulations of material extend, decreasing, from the heel area (3) to the area of the front part of the foot.

2. Ankle pad (8) according to claim 1,
**characterized in that**
each of its profiled inlays (9) possesses a relief which is inclined from the area of the Achilles tendon (2) towards the inner side (6) of the foot (15), and in that this relief has a convex curvature, its convex inner side (6) facing the ankle joint (14) and its outward facing edges running out, flattening, towards the outer sides (5) of the inlays.

3. Ankle pad (8) according to claims 1 and 2,
**characterized in that**
each profiled inlay (9) is provided in its area extending up to the heel area (3) of the foot (15) with a protruding projection (7).

4. Ankle pad (8) according to claim 1,
**characterized in that**
the viscoelastic material of each profiled inlay (9) is a silicone material and that the hardness of this material differs along the profile of the profiled inlay (9), starting in the area of the Achilles tendon (2) and running out towards the inner side of the foot (15), such that around the area of the ankle (16, 17) the material is less hard, whereas in the remaining area of the profiled inlay (9), it is of a higher hardness.

5. Ankle pad (8) according to claim 4,
**characterized in that**
one of the two profiled inlays (9) as a whole is made of a less hard viscoelastic material than the other profiled inlay (9).

6. Ankle pad (8) according to claim 1,
**characterized in that**
at least one of the profiled inlays (9) is provided for further stabilization of the ankle joint (14) with an L-shaped reinforcing element (13), which is arranged along an anatomically recessed portion (furrow) between the ankle (16) and the heel area (3).

7. Ankle pad (8) according to claim 4,
**characterized in that**
the viscoelastic material of each profiled inlay (9) is a PVC.

8. Ankle pad (8) according to claim 4,
**characterized in that**
the viscoelastic material of each profiled inlay (9) is an addition silicone.

9. Ankle pad (8) according to one or several of claims 1-8,
**characterized in that**
the respective edges (10) situated in the sole area (4) of the two profiled inlays (9) are connected with each other via a link (11), and in that this link lies, when the ankle pad (8) is being worn, under the sole between the heel area (3) and the ball of the foot.

10. Ankle pad (8) according to claim 9,
**characterized in that**
the link (11) extends over at least one third of the length of the edge (10) facing the sole, of the profiled inlay (9).

11. Ankle pad (8) according to at least one of claims 1-10, wherein the profiled inlays (9) are held by an elastic, form-knitted bandage around the respective ankle (16, 17) of the ankle joint (14),
**characterized in that**
the compressive force of their wall parts (surfaces 18, 19) acting upon the ankle joint (14) depends on the degree of elasticity of the bandage.

12. Ankle pad (8) according to at least one of claims 1-10,
**characterized in that**
the profiled inlays (9) are fixed to the inner sides of an ankle-boot-shaped shaft (12) and in that the compressive force of their wall parts (surfaces 18, 19) acting upon the ankle joint (14) is a function of the tightness of the lacing.

## Revendications

1. Pelote malléolaire (8) pour produire une compression intermittent dans la zone de l'articulation tibio-tarsienne (14) d'un pied (15), comportant
un insert profilé (9) fabriqué d'un matériau visco-élastique pour chaque côté de l'articulation tibio-tarsienne (14), chaque insert profilé (9) étant attaché à un élément tendeur qui le tient et le serre contre l'articulation tibio-tarsienne (14) et l'insert profilé respectif recouvrant, au moins partiellement, la malléole (16, 17) y appartenant de l'articulation tibio-tarsienne (14),
**caractérisée en ce**
que chaque insert profilé (9) de la pelote malléolaire (8) a la forme d'une languette et ses surfaces (18, 19) en regard de l'articulation tibio-tarsienne (14) ont une courbure convexe et recouvrent, en plus de la zone en relief entre la malléole (16, 17) d'un côté et le zone du tendon d'Achille (2) d'un autre côté, la zone du talon (3) et la zone de la plante du pied (4) sur une large surface, ainsi que, du moins partiellement, la zone de la malléole (1),
et en ce que chaque insert profilé (9) possède des éléments de paroi enrobables dans l'insert sur son côté en regard de l'articulation tibio-tarsienne (14) dans les parties anatomiquement creuses respectives de la zone en forme de sillon autour de la malléole (16, 17) respective, afin de remplir ces parties creuses de cette zone, les éléments de parois étant enrobées dans ces parties lorsque la pelote malléolaire (8) est portée et lorsqu'elle est en contact avec l'articulation tibio-tarsienne (14) en maintenant là la malléole elle-même ainsi que la zone autour de la malléole (16, 17) remplies et comprimées, et suivant là, de plus, le relief anatomique de l'articulation tibio-tarsienne (14) moyennant une accumulation plus ou moins importante du matériau de l'insert profilé (9), de telle sorte que ces accumulations de matériau s'étendent, en diminuant, de la zone du talon (3) jusqu'à la zone de la partie de devant du pied.

2. Pelote malléolaire (8) selon la revendication 1,
**caractérisée en ce**
chacun de ses inserts profilés (9) possède un relief qui est incliné de la zone du tendon d'Achille (2) vers le côté intérieur (6) du pied (15), et en ce que ce relief a une courbure convexe, son côté intérieur convexe (6) se trouvant en regard de l'articulation tibio-tarsienne (14) et ses bords en regard vers l'extérieur se terminant, tout en s'aplatissant, vers les côtés extérieurs (5) des inserts.

3. Pelote malléolaire (8) selon les revendications 1 et 2,
**caractérisée en ce**
chaque insert profilé (9) est pourvu dans sa zone s'étenddant jusqu'à la zone du talon (3) du pied (15) d'une projecture (7) faisant saillie.

4. Pelote malléolaire (8) selon la revendication 1,
**caractérisée en ce**
le matériau visco-élastique de chaque insert profilé (9) est un matériau silicone et que la dureté de ce matériau diffère le long du profil de l'insert profilé (9), commençant dans la zone du tendon d'Achille (2) et se terminant vers le côté intérieur du pied (15), de façon qu'autour de la zone de la malléole (16, 17) le matériau est moins dur, tandis que dans le reste de la zone de l'insert profilé (9), il a une plus grande dureté.

5. Pelote malléolaire (8) selon la revendication 4,
**caractérisée en ce**
l'un des deux inserts profilés (9) dans son ensemble est fabriqué d'un matériau visco élastique moins dur que l'autre insert profilé (9).

6. Pelote malléolaire (8) selon la revendication 1,
**caractérisée en ce**
qu'au moins l'un des inserts profilés (9) est pourvu, pour encore mieux stabiliser l'articulation tibio-tarsienne (14), d'un élément de renforcement (13) en forme d'une L qui se trouve le long d'une partie anatomiquement creuse (sillon) entre la malléole (16) et la zone du talon (3).

7. Pelote malléolaire (8) selon la revendication 4,
**caractérisée en ce**
que le matériau visco-élastique de chaque insert profilé (9) est un PVC.

8. Pelote malléolaire (8) selon la revendication 4,
**caractérisée en ce**
que le matériau visco-élastique de chaque insert profilé (9) est une silicone d'addition.

9. Pelote malléolaire (8) selon une ou plusieurs des revendications 1-8,
**caractérisée en ce**
les bords (10) respectifs situés dans la zone de la plante du pied (4) des deux inserts profilés (9) sont connectés l'un avec l'autre au moyen d'une traverse (11), et en ce que cette traverse, lorsque la pelote malléolaire (8) est portée, se trouve, au-dessous de la plante du pied entre la zone du talon (3) et l'éminence du gros orteil.

10. Pelote malléolaire (8) selon la revendication 9,
**caractérisée en ce**
la traverse (11) s'étend sur du moins un tiers de la longueur du bord (10) en regard de la plante du pied, de l' insert profilé (9).

11. Pelote malléolaire (8) selon du moins l'un des revendications 1-10, les inserts profilés (9) étant tenues par un bandage élastique, tricoté de forme, autour de la malléole (16, 17) respective de l'articulation tibio-tarsienne (14),
**caractérisée en ce**
la force de compression de leurs éléments de paroi (surfaces 18, 19) agissant sur l'articulation tibio-tarsienne (14) est dépendant du degré d'élasticité du bandage.

12. Pelote malléolaire (8) selon du moins l'un des revendications 1-10,
**caractérisée en ce**
les inserts profilés (9) sont fixés aux côtés intérieurs d'une tige (12) en forme de demi-botte et en ce que la force de compression de leurs éléments de paroi (surfaces 18, 19) agissant sur l'articulation tibio-tarsienne (14) est dépendant de la fermeté du laçage.
